# EUROPÄISCHE PATENTANMELDUNG

(11) **EP 4 656 731 A1**
(43) Veröffentlichungstag der Anmeldung: **03.12.2025**
(21) Anmeldenummer: 24178863.7
(22) Anmeldetag: 29.05.2024
(51) Int. Cl.: C12P 7/58, C12N 9/04, C12N 9/02, C12P 19/36

(54) **VERFAHREN ZUR HERSTELLUNG VON GALACTARSÄURE**

(71) Anmelder: Annikki GmbH, 8074 Raaba-Grambach (AT)
(72) Erfinder: Staunig, Nicole, 8074 Raaba-Grambach (AT); Frühwirt, Philipp, 8074 Raaba-Grambach (AT)
(74) Vertreter: Schwarz & Partner Patentanwälte GmbH

(57) **Zusammenfassung**

Verfahren zur Herstellung von Galactarsäure, indem Galacturonsäure in einer wässerigen Lösung *in vitro* mit einer Dehydrogenase mit NAD(P)⁺ als Kofaktor unter Bildung von reduziertem Kofaktor NAD(P)H oxidiert wird, dadurch gekennzeichnet, dass der reduzierte Kofaktor NAD(P)H mit einer NAD(P)H-Oxidase oxidiert wird.

## Beschreibung

Die vorliegende Erfindung betrifft ein enzymatisches Verfahren zur Herstellung der Dicarbonsäure Galactarsäure.

### Hintergrund der Erfindung

Galactarsäure, auch bekannt als Mucinsäure oder Schleimsäure, ist eine Zuckerdicarbonsäure (Aladarsäure). Sie dient als Baustein für Polykondensate (z.B. Polyamide), für SBAPs (Sugar-based amphiphilic polymers), die als Drug Carrier für hydrophobe Krebswirkstoffe oder Gentherapeutika oder zur Behandlung von Atherosklerose verwendet werden können, für Metal Organic Frameworks (MOFs) (Sakuta & Nakamura, 2019) sowie zur Herstellung von Plattformchemikalien Adipinsäure (Li et al., 2014) oder 2,5-Furandicarbonsäure (US 9701652 B2; US 9994539 B2).

Galactarsäure und ihr Anion Galactarat kommen in der Natur beispielsweise in Früchten (Anet & Reynolds, 1954) oder in Zuckerrüben (Stark et al., 1950) nur in geringen Mengen vor, was eine synthetische Herstellung unumgänglich macht. So kann D-Galactose mittels Salpetersäure zu Galactarsäure oxidiert werden (Pigman et al., 1949), jedoch führt das Verfahren zu niedrigen Umsätzen sowie zur Bildung von Nebenprodukten und Stickoxiden (Sakuta & Nakamura, 2019).

Andere Verfahren setzen auf die Vorstufe D-Galacturonsäure (oder ihr Anion D-Galacturonat), die anstelle von zwei terminalen Carbonsäuren nur eine Carbonsäure- und eine Aldehyd-Funktion aufweist. D-Galacturonsäure kommt in der Natur als Hauptbestandteil des Heteropolysaccharids Pektin vor. Pektin findet sich in Zellwänden von Pflanzenzellen - besonders in Früchten wie Äpfeln und Zitrusfrüchten, in Zuckerrüben-Pulpe oder in Kartoffelschalen. Insgesamt stehen mehr als 1,5 Mt D-Galacturonsäure (gebunden in Pektin) weltweit zur Verfügung (van der Klis et al., 2017; Roman-Benn et al., 2023).

Je nach Aufbau des Pektins können Homogalacturonan, Rhamnogalacturonan I, Rhamnogalacturonan II und Xylogalacturonan unterschieden werden. Durch die Einwirkung der Pektinasen, einer Gruppe von Pektin-abbauenden Enzymen wie z.B. der Polygalacturonase, können die Monomere freigesetzt werden (Kuivanen et al., 2019; Roman-Benn et al., 2023).

Die Aldehyd-Funktion in D-Galacturonsäure kann entweder chemisch-katalytisch oder biokatalytisch (*in vivo* oder *in vitro*) oxidiert werden.

Rautiainen et al. (2015) nutzten einen Au/Al₂O₃-Katalysator zur Oxidation von D-Galacturonsäure zu Galactarsäure bei pH 8 - 10 und 40 - 60 °C mit Luftsauerstoff. EP 2836498 B1 und EP 3204155 B1 beschreiben ähnliche Verfahren und geben Zuckerrübenpulpe-Hydrolysate und Zitrusfrucht-Hydrolysate als Quellen für D-Galacturonsäure an.

Ebenfalls einen Gold-Katalysator zur Oxidation nutzten van der Klis et al. (2018), die eine höhere Produktivität der Oxidation durch Umstieg von einem Batch-Reaktor (siehe dazu van der Klis et al. (2013)) zu einem Plug Flow-Festbettreaktor erzielen konnten. Da das Verfahren bei erhöhten pH-Werten (pH ≥ 9) durchgeführt wird, wurden Spuren von 5-Keto-L-Galactonat, welches durch die basenkatalysierte Isomerisierung von D-Galacturonat entsteht, gefunden. Als weiteres Nebenprodukt wurde von den Autoren D-Talarsäure/D-Talarat, als Oxidationsprodukt von D-Taluronsäure/D-Taluronat (C2-Epimer von D-Galacturonat) postuliert (van der Klis et al., 2018).

Ein genereller Nachteil der hier angeführten Verfahren ist der Bedarf an Gold als Katalysator, das zum einen teuer ist und zum anderen nur begrenzt in der Natur vorkommt.

Als Alternative bieten sich biokatalytische Prozesse an, die hochselektiv unter milden Reaktionsbedingungen ablaufen und bioabbaubare Katalysatoren wie Zellen oder Enzyme verwenden.

Mojzita et al. (2010) verwendeten engineerte Pilzstämme (*Hypocrea jecorina* und *Aspergillus niger*) zur Oxidation von D-Galacturonat zu Galactarat. Dazu wurde das Gen, welches die D-Galacturonat-Reduktase (D-Galacturonat → L-Galactonat) kodiert, deletiert und das bakterielle udh-Gen, welches eine NAD-abhängige D-Galacturonat-Dehydrogenase (eine Uronat-Dehydrogenase (UDH), EC 1.1.1.203) kodiert, eingeführt. Die resultierenden Stämme konnten D-Galacturonat oxidieren, wobei der *H. jecorina-Stamm* Galactarat mit höheren Umsätzen bilden kann (15,8 g/l nach 24 h). Galactarsäure konnte durch Ansäuern aus den Kultur-Überständen in hoher Reinheit (> 94%) isoliert werden.

Die Expression der UDH aus *Agrobacterium tumefaciens* CS58 (*Rhizobium radiobacter*) in modifizierten (Deletion der D-Galacturonat-Reduktase) *Aspergillus* sp. oder *Hypocrea* sp. wird in US 8895273 B2 beschrieben. Mit einem *H. jecorina* (= *Trichoderma* reesei)-Mutanten konnten 10 g/l D-Galacturonat zu 1-1,3 g/l Galactarat oxidiert werden, wobei 10 g/l D-Xylose für das Wachstum der Stämme zugesetzt wurde.

Die direkte Umwandlung von Pektin zu Galactarat mit dem modifizierten Pilz *Trichoderma reesei* wurde von Paasikallio et al. (2017) beschrieben. 29,2 g/l Pektin konnten im 10L-Maßstab in 185 h zu 21 g/l Galactarsäure umgesetzt werden, wobei 85% des Produkts isoliert werden konnten. Tamminen et al. (2022) verwendeten ebenfalls einen *Trichoderma* sp.-Transformanten, der bis zu 53 g/l Galactarsäure in einem Fed-Batch-Reaktor in 300 h produzierte.

Protzko et al. (2018) nutzten einen engineerten *Saccharomyces cerevisiae* mit exprimiertem GatA (A. *niger* D-Galacturonat-Transporter) und UDH, um D-Galacturonat aus Zitrusschalen-Abfall zu Galactarat zu oxidieren (Titer 8 g/l nach 80 h), wobei noch zusätzlich D-Glucose zugegeben wurde. Das Verfahren ist auch in US 11332723 B2 beschrieben.

Analog zu Mojzita et al. (2010) engineerten Vidgren et al. (2020) zwei marine Pilze (*Trichoderma* sp. und *Coniochaeta* sp.) für die Produktion von Galactarat. Der Transformant *Trichoderma* sp. LF328 T2 produzierte bis zu 25 g/l Galactarat in 200 h (mit D-Glucose als Kosubstrat). Ein *Coniochaeta* sp.-Transformant wiederum konnte Galactarat direkt aus Pektin herstellen, der D-Galacturonat-Metabolismus wurde aber nicht komplett zum Erliegen gebracht.

Modifizierte Mikroorganismen zur Produktion von Galactarat aus D-Galacturonat werden des Weiteren in US 10982239 B2, EP 3486323 A1 sowie in WO 2010/072902 A1 genannt.

Auch enzymatische Verfahren zur Herstellung von Galactarat sind bekannt.

Wagner und Hollmann (1976) beschreiben einen enzymatischen Assay zur spektrophotometrischen Bestimmung von (freier und konjugierter) D-Glucuronsäure und D-Galacturonsäure, wobei als Enzym eine UDH aus *Pseudomonas syringae* verwendet wurde.

Der Artikel von Chang und Feingold (1969) beschreibt die Herstellung von Galactarsäure mit einer Hexuronsäure-Dehydrogenase aus *Agrobacterium tumefaciens.* Dazu wurden 7 mM (1,4 g/l) D-Galacturonsäure mit dem Enzym angesetzt - zur Regeneration des Kofaktors diente eine Lactat-Dehydrogenase aus Kaninchenmuskel (EC 1.1.1.27) mit Kaliumpyruvat (30 mM; 4,3 Äquivalente bezogen auf D-Galacturonsäure) als Kosubstrat. Galactarsäure konnte als Produkt nach Aktivkohlefiltration, lonenaustausch-Chromatographie und anschließender Kristallisation isoliert werden - die Produkt-Ausbeute wurde im Artikel nicht angegeben.

Zur Herstellung der D-Glucarsäure (C3-Epimer der Galactarsäure) sind ebenfalls enzymatische Verfahren bekannt.

Su et al. (2019) beschreiben die Umsetzung von 50 mM Saccharose zu 34,8 mM D-Glucarsäure *in vitro* in 70 h mit einem aus sieben Enzymen bestehenden Kaskadensystem. Die im vorletzten Schritt entstehende D-Glucuronsäure wird dabei mittels UDH aus *Agrobacterium tumefaciens* und NAD⁺ zu D-Glucarsäure oxidiert, wobei das dabei entstehende NADH mit einer NADH-Oxidase aus *Lactobacillus rhamnosus* oxidiert wird.

Petroll et al. (2020) verwendeten eine ähnliche Enzymkaskade bestehend aus sechs Enzymen, um Glucose-1-phosphat (G1P), das mittels Phosphorylasen entweder aus Stärke, Saccharose oder Cellulose gewonnen werden kann, ebenfalls *in vitro* zu D-Glucarsäure umzusetzen. Der letzte Schritt der Kaskade umfasste ebenfalls die Oxidation mittels UDH (aus *Fulvimarina pelagi*) sowie NADH-Oxidase aus *Lactobacillus rhamnosus* zur Kofaktor-Regeneration. Auf diese Weise konnten 40 mM G1P in 10 h zu 8,1 mM D-Glucarsäure umgesetzt werden.

Neben der UDH gibt es weitere Enzyme, die zur Oxidation von D-Galacturonsäure eingesetzt werden können.

Sakuta et al. (2016) entwickelten eine Elektrode mit immobilisierter Pyrrolochinolinchinon-abhängiger Glucose-Dehydrogenase (PQQ-GDH), welche die Oxidation von D-Galacturonat zu Galactarat bioelektrochemisch bewerkstelligt. Diese Elektrode wurde auch von Nakagawa et al. (2022) zur Umsetzung von D-Galacturonat zu Galactarat in einer enzymatischen Biobrennstoffzelle verwendet. Nachteilig ist die Verwendung des Farbstoffs Methylengrün als Elektronakzeptor sowie die Bildung von Galactarolacton als Nebenprodukt (Vastano et al., 2019).

Auch die Glucose-Oxidase aus *A. niger* zeigt Uronsäure-Oxidase-Aktivität, wie Kobayashi et al. (1999) zeigen konnten, allerdings werden neben den Uronsäuren auch Aldosen (D-Glucose, D-Galactose, D-Mannose und D-Xylose) oxidiert.

Vastano et al. (2019) testeten drei Enzyme zur Oxidation von D-Galacturonat zu Galactarat: eine kommerzielle Laccase, eine Laccase von *Myceliophthora thermophila* (beide mit dem stabilisierten Radikal 2,2,6,6-Tetramethylpiperidinyloxyl (TEMPO) als Mediator) sowie PQQ-GDH (mit Methylengrün als Mediator). Bei allen Enzymen konnten ähnliche Umsätze (ca. 40% nach 12 d bei bis zu 500 mM (97 g/l) Substratkonzentration) beobachtet werden.

Aus der Shamouti-Orange (*Citrus sinensis* L. Osbeck) ist die Uronsäure-Oxidase bekannt, die D-Galacturonsäure und D-Glucuronsäure in die entsprechenden Aldarsäuren überführen kann (Riov, 1975).

Boverio et al. (2023) charakterisierten ebenfalls eine Uronsäure-Oxidase aus *Citrus sinensis,* mit der 0,35 w% D-Galacturonsäure in 6 h komplett zu Galactarsäure oxidiert werden kann. Ähnliche Umsätze wurden auch für eine Mischung von D-Galacturonsäure (0,32 w%) und L-Arabinose, D-Galactose und D-Glucose (in Summe 0,11 w%) beobachtet, welche durch enzymatische Hydrolyse von Pektin aus einer Zuckerrüben-Pulpe erhalten worden war. L-Arabinose, D-Galactose und D-Glucose wurden nicht von der Uronsäure-Oxidase oxidiert.

Als Nebenprodukt der Uronsäure-Oxidation entsteht Wasserstoffperoxid, welches schädlich für Enzyme ist und daher entfernt werden muss (z.B. mittels einer Katalase), was einen großen Nachteil darstellt.

Hier setzt nun die Aufgabe der vorliegenden Erfindung an und setzt sich zum Ziel, ein alternatives Verfahren zur Herstellung von Galactarsäure aus D-Galacturonsäure zur Verfügung zu stellen.

### Detaillierte Beschreibung der Erfindung

Die Aufgabe wird erfindungsgemäß gelöst, indem Galacturonsäure in einer wässerigen Lösung *in vitro* mit einer Dehydrogenase mit NAD(P)⁺ als Kofaktor unter Bildung von reduziertem Kofaktor NAD(P)H oxidiert wird, und ist dadurch gekennzeichnet, dass der reduzierte Kofaktor NAD(P)H mit einer NAD(P)H-Oxidase oxidiert und damit regeneriert wird.

Es hat sich gezeigt, dass der Einsatz von isolierten Dehydrogenasen sehr wohl eine gute Alternative zum Einsatz von Oxidasen darstellt (Boverio et al. 2023), wenn man diese mit einem geeigneten Kofaktor-Regenerationssystem wie z.B. einer NAD(P)H-Oxidase kombiniert.

Als Dehydrogenase wird bevorzugt eine Uronsäure/Uronat-Dehydrogenase (EC 1.1.1.203) eingesetzt. Geeignete Uronsäure-Dehydrogenasen sind beispielsweise aus den Organismen *Pseudomonas putida* oder *Streptomyces viridochromogenes* erhältlich (siehe Tabelle 1).

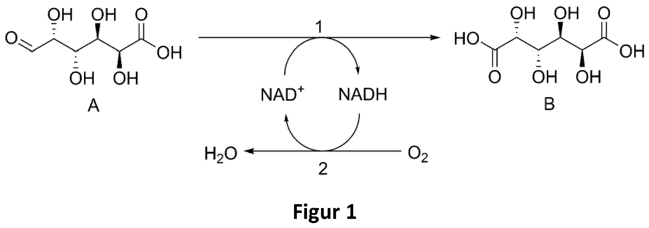

Das erfindungsgemäße Verfahren ist in der beiliegenden Figur 1 schematisch dargestellt, wobei die Galacturonsäure in der D-Form dargestellt ist. Die Bezeichnung A in Figur 1 steht dabei für D-Galacturonsäure, B für Galactarsäure, 1 für Uronsäure-Uronat-Dehydrogenase und 2 für NADH-Oxidase.

Die besonders bevorzugte Konzentration von Galacturonsäure liegt zwischen 50 und 200 g/l.

Der besonders bevorzugte Temperaturbereich für das erfindungsgemäße Verfahren liegt zwischen 20 und 40 °C.

Der besonders bevorzugte pH-Bereich liegt zwischen 5 und 9.

D-Galacturonsäure kann beispielsweise durch Hydrolyse von Pektin hergestellt werden, welches beispielsweise aus Zuckerrüben-Pulpe, Kartoffelschalen, Apfeltrester oder Zitrus-Schalen gewonnen werden kann, wobei letzteres bevorzugt eingesetzt wird.

Die Abtrennung der Enzyme kann z.B. durch Zentrifugieren oder Ultrafiltration bewerkstelligt werden.

Bei einer weiteren, bevorzugten Variante des erfindungsgemäßen Verfahrens liegen die Enzyme in einer Suspension, im Homogenat und/oder im Lysat der entsprechenden, sie bildenden Zellen vor, wobei Lysate besonders bevorzugt sind.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund der Entfernung von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme* & *Herstellung der Lysate* für Details).

In einer weiteren Variante können die Enzyme auch mit einem wasserlöslichen Polymer wie Polyethylenglycol am N-Terminus modifiziert, in oder auf einer festen Matrix immobilisiert oder Teil eines Fusionsproteins sein.

Die zur Kofaktor-Regeneration eingesetzte NAD(P)H-Oxidase kann aus einer der Gruppen EC 1.6.3.1 (NAD(P)H-Oxidase (H₂O₂-bildend)), EC 1.6.3.2 (NAD(P)H-Oxidase (H₂O-bildend)), EC 1.6.3.3 (NADH-Oxidase (H₂O₂-bildend)) sowie EC 1.6.3.4 (NADH-Oxidase (H₂O-bildend)) stammen, wobei die H₂O-bildenden Klassen besonders bevorzugt sind.

Eine besonders bevorzugt eingesetzte H₂O-bildende NAD(P)H-Oxidase zur Oxidation von NAD(P)H zu NAD(P)⁺ umfasst oder besteht vorzugsweise aus eine Aminosäuresequenz, die ausgewählt ist aus der Gruppe bestehend aus:
i) einer Aminosäuresequenz, die eine Identität zu SEQ ID Nr. 2 oder SEQ ID Nr. 4 von mindestens 80% aufweist,
ii) einer Aminosäuresequenz, die durch eine Nukleinsäure kodiert wird, die eine Identität zu SEQ ID Nr. 1 oder SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 oder SEQ ID Nr. 3 bindet.

SEQ ID Nr. 1: 
ATGAAAGTAGTAGTAGTAGGCTGTACACATGCAGGAACAGCGGCAGTTAAGACGATTTTAAATGAACATCCA
GATGCATCAGTATCAGTATATGAGCGTAATGACAATGTCTCATTTCTATCTTGTGGGATTGCGTTGTATGTTGG
TGGAGTTGTGAAAGATCCTGCAGGTTTGTTTTATTCAAGTCCAGAAGAACTTGCATCAATGGGCGCGAAAATT
AACATGGAACACAATGTGAAAAATATAGATAATGAGAATAAGGTCGTAGTAATTGAGAATTTAAAAACAGGC
GAAACATTTGAAGAAAGCTATGATAAGTTGGTAATGACAACTGGATCATGGCCAATTATTCCTCCAATTGATGG
AATCAATAGTGAAAATATTCTTTTGTGTAAAAACTATAACCAAGCAAATGAAATTATTAAAGAATCAAAAAATG
CTAAAAAGATTGTCATTGTTGGTGGTGGCTATATTGGAATTGAATTAGTTGAGGCATTTGCAGAATCTGGCAA
GCAAGTGACGCTAGTTGATGGATTAGATCGTATTTTAAACAAATATTTAGATGCTGAATTCACTTCTGTTTTAGA
GCATGATTTACAAGAAAGAGGCGTTACGCTAGCTTTAAACCAAACCGTCGAGAAATTTGTTGCCAATGAATCA
GGTGCTGTGACAGCTGTGAAAACACCAGTTGGAGAATATGAGGCTGATTTAGTTATTTTATGTGTTGGATTTAA
 
ACCAAATACTGATTTGTTGAAGGATAAAGTAGAGATGTTGCCAAATGGTGCCATCGTAGTGGATGAATATATG
AGAACAAGCGATGAAGCGATTTTTGCTGCTGGCGATAGTTGCGCGGTTCATTATAATCCAACTGGAGGCTCTG
CGTATATTCCGTTAGCTACAAATGCAGTTAGAATGGGAGCTTTAGTTGGGAAAAATATTGTTTCTCCAACAGTT
AAATATCGTGGCACGCAAGCAACTTCTGGTTTATATTTATTTGGTTTTAATATAGGTTCAACCGGATTGACTGAA
AATAGCGCTCCTCATTTTGGCGTAGAGGTTCGTTCAGTAGTTGTAGAAGATAATTATCGTCCAGAGTTTATGCC
GACAACAGAGAAAGTAACGATGAAATTAGTTTATGAAGTAGGAACGAATCGGATTGTTGGAGGTCAAATCAT
GTCAAAATATGATGTGACACAATCTGCCAATACGTTATCTTTATGTGTTCAAAATAAAATGACGATTGAGGATT
TGGCTTATGTAGATTTCTTCTTCCAACCTCACTTTGATCGTCCTTGGAACTATTTAAATATTTTAGCGCAAGCAGC
TGTTGAGCAAGAGCGTAAACTAGCAAAATAA
SEQ ID Nr. 2: 
MKVVVVGCTHAGTAAVKTILNEHPDASVSVYERNDNVSFLSCGIALYVGGVVKDPAGLFYSSPEELASMGAKINME
HNVKNIDNENKVVVIENLKTGETFEESYDKLVMTTGSWPIIPPIDGINSENILLCKNYNQANEIIKESKNAKKIVIVGG
GYIGIELVEAFAESGKQVTLVDGLDRILNKYLDAEFTSVLEHDLQERGVTLALNQTVEKFVANESGAVTAVKTPVGEY
EADLVILCVGFKPNTDLLKDKVEMLPNGAIVVDEYMRTSDEAIFAAGDSCAVHYNPTGGSAYIPLATNAVRMGALV
GKNIVSPTVKYRGTQATSGLYLFGFNIGSTGLTENSAPHFGVEVRSVVVEDNYRPEFMPTTEKVTMKLVYEVGTNRI
VGGQIMSKYDVTQSANTLSLCVQNKMTIEDLAYVDFFFQPHFDRPWNYLNILAQAAVEQERKLAK
SEQ ID Nr. 3: 
ATGAGCAAAATTGTTATCGTGGGTGCAAATCATGCAGGCACCGCAGCAATTAATACCATTCTGGATAATTATG
GCAGCGAAAATGAAGTGGTTGTGTTTGATCAGAATAGCAACATTAGCTTTCTGGGTTGTGGTATGGCACTGTG
GATTGGTAAACAAATTAGCGGTCCGCAGGGTCTGTTTTATGCAGATAAAGAAAGCCTGGAAGCAAAAGGTGC
CAAAATCTATATGGAAAGTCCGGTTACCGCCATTGATTATGATGCAAAACGTGTTACCGCACTGGTTAATGGTC
AAGAACATGTTGAAAGCTACGAGAAACTGATTCTGGCAACCGGTAGCACCCCGATTCTGCCTCCGATTAAAGG
TGCAGCCATTAAAGAAGGTAGTCGCGATTTTGAAGCAACCCTGAAAAATCTGCAGTTCGTGAAACTGTATCAG
AATGCCGAAGATGTGATTAACAAACTGCAGGATAAAAGCCAGAATCTGAATCGTATTGCAGTTGTTGGTGCAG
GTTATATTGGTGTTGAACTGGCAGAAGCATTTAAACGTCTGGGTAAAGAAGTGATTCTGATTGACGTTGTTGAT
ACCTGTCTGGCAGGTTATTATGATCAGGATCTGAGCGAAATGATGCGTCAGAATCTGGAAGATCATGGTATCG
AACTGGCATTTGGTGAAACCGTTAAAGCAATTGAAGGTGATGGTAAAGTGGAACGTATTGTTACCGATAAAGC
AAGCCATGATGTGGATATGGTTATTCTGGCAGTTGGTTTTCGTCCGAATACAGCACTGGGTAATGCAAAACTG
AAAACCTTTCGTAATGGTGCCTTTCTGGTGGATAAAAAACAAGAAACCAGCATCCCGGATGTTTATGCAATTGG
TGATTGTGCAACCGTGTATGATAATGCCATTAACGACACCAACTATATTGCACTGGCAAGCAATGCACTGCGTA
GCGGTATTGTTGCAGGTCATAATGCAGCCGGTCATAAACTGGAAAGTCTGGGTGTTCAGGGTAGCAATGGTAT
TTCAATTTTTGGCCTGAATATGGTTAGCACCGGTCTGACCCAAGAAAAAGCCAAACGTTTTGGTTATAATCCGG
AAGTTACCGCCTTTACCGATTTTCAGAAAGCCAGCTTTATCGAGCATGATAACTATCCGGTTACGCTGAAAATT
GTGTATGACAAAGATAGCCGTCTGGTTCTGGGTGCACAGATGGCCAGCAAAGAAGATATGAGCATGGGTATT
 
CACATGTTTAGCCTGGCCATTCAAGAGAAAGTTACCATTGAACGTCTGGCCCTGCTGGATTATTTCTTTCTGCCG
CATTTTAATCAGCCGTACAACTATATGACCAAAGCAGCACTGAAAGCCAAATAA
SEQ ID Nr. 4:

MSKIVIVGANHAGTAAINTILDNYGSENEVVVFDQNSNISFLGCGMALWIGKQISGPQGLFYADKESLEAKGAKIY
MESPVTAIDYDAKRVTALVNGQEHVESYEKLILATGSTPILPPIKGAAIKEGSRDFEATLKNLQFVKLYQNAEDVINKL
QDKSQNLNRIAVVGAGYIGVELAEAFKRLGKEVILIDVVDTCLAGYYDQDLSEMMRQNLEDHGIELAFGETVKAIEG
DGKVERIVTDKASHDVDMVILAVGFRPNTALGNAKLKTFRNGAFLVDKKQETSIPDVYAIGDCATVYDNAINDTNYI
ALASNALRSGIVAGHNAAGHKLESLGVQGSNGISIFGLNMVSTGLTQEKAKRFGYNPEVTAFTDFQKASFIEHDNYP
VTLKIVYDKDSRLVLGAQMASKEDMSMGIHMFSLAIQEKVTIERLALLDYFFLPHFNQPYNYMTKAALKAK

Die bevorzugt verwendete HjO-bildende NAD(P)H-Oxidase zur Oxidation von NAD(P)H zu NAD(P)⁺ umfasst oder besteht vorzugsweise aus einer Aminosäuresequenz, die eine Identität zu SEQ ID Nr. 2 oder SEQ ID Nr. 4 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die H₂O-bildende NAD(P)H-Oxidase die Aminosäuresequenz SEQ ID Nr. 2 oder SEQ ID Nr. 4 oder besteht aus dieser.

Alternativ umfasst die H₂O-bildende NAD(P)H-Oxidase zur Oxidation von NAD(P)H zu NAD(P)⁺ vorzugsweise eine Aminosäuresequenz, die durch eine Nukleinsäure kodiert wird, die eine Identität zu SEQ ID Nr. 1 oder SEQ ID Nr. 3 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die HjO-bildende NAD(P)H-Oxidase kodiert, die Nukleinsäuresequenz SEQ ID Nr. 1 oder SEQ ID Nr. 3 oder besteht aus dieser.

Ein weiterer Aspekt der vorliegenden Erfindung betrifft die die Verwendung einer H₂O-bildenden NAD(P)H-Oxidase zur Oxidation von NAD(P)H zu NAD(P)⁺, welche eine Aminosäuresequenz umfasst oder daraus besteht, die ausgewählt ist aus der Gruppe bestehend aus:
i) einer Aminosäuresequenz, die eine Identität zu SEQ ID Nr. 2 oder SEQ ID Nr. 4 von mindestens 80% aufweist,
ii) einer Aminosäuresequenz, die durch eine Nukleinsäure kodiert wird, die eine Identität zu SEQ ID Nr. 1 oder SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 oder SEQ ID Nr. 3 bindet.

### Materialien

D-Galacturonsäure-Monohydrat, Galactarsäure, D-Glucose, sowie D-Xylose wurden von Sigma-Aldrich, Pektinase aus *Aspergillus niger* wurde von TCI, Methanol, Acetonitril, L-Rhamnose-Monohydrat sowie D-Galactose, NAD⁺, NADH-Dinatriumsalz, NADP⁺-Dinatriumsalz und NADPH-Tetranatriumsalz wurden von PanReac AppliChem (ITW Reagents), L-Arabinose, IPTG (Isopropyl-β-D-thiogalactopyranosid), Kaliumdihydrogenphosphat, di-Kaliumhydrogenphosphat sowie Natriumdodecylsulfat (SDS) wurden von Carl Roth und Triethanolamin (TEA) wurde von Chem-Lab NV bezogen.

### Produktion der Enzyme & Herstellung der Lysate

### Allgemeines zur Expression von rekombinanten Enzymen in E. coli

Für die rekombinante Enzymproduktion in einem *Escherichia coli*-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E. coli*-Zellen Top10F transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTGinduzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optische Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels Sonifier-Aufschlusses

Zur Herstellung einer Zell-Suspension wurde das nach obigen Verfahren hergestellte Zellpellet in einem geeigneten Gefäß eingewogen und mit Puffer und Lysozym (finale Konzentration 0,5 mg/ml) versetzt (z.B. Triethanolamin (TEA) - HCl) und unter Rührung gelöst. Der Massenanteil an Biomasse beträgt üblicherweise 20%, der Rest entfällt auf den Puffer.

Zum Zell-Aufschluss wurde ein Branson Sonifier 450 verwendet. Die Suspension wurde dreimal mit je 15 Ultraschall-Stößen (Einstellungen am Gerät: Timer = 15; Duty Cycle = 50; Output Control = 3 - 5) behandelt.

Das erhaltene Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Enzymklassen und Spenderorganismen für die in den Beispielen verwendeten Enzyme.**

| **Enzymtyp (EC-Klasse)** | **katalysierte Reaktion** | **Spenderorganismus** | **Literatur** |
|---|---|---|---|
| Uronat-Dehydrogenase I (UDH; EC 1.1.1.203) | D-Galacturonat → Galactarat | *Pseudomonas putida* | (NCBI Protein Database: WP_134697446.1); (Yoon et al., 2009) |
| Uronat-Dehydrogenase II (UDH; EC 1.1.1.203) | D-Galacturonat → Galactarat | *Streptomyces viridochromogenes* | (NCBI Protein Database: WP _003989401.1); (Pick et al., 2015) |
| NADH-Oxidase I (EC 1.6.3.4) | NADH → NAD⁺ | *Carnobacterium divergens* | SEQ ID Nr. 2 |
| NADH-Oxidase II (EC 1.6.3.4) | NADH → NAD⁺ | *Streptococcus mutans* | (Matsumoto et al., 1996); SEQ ID Nr. 4 |
| Pektinase (kommerziell) | Pektin → D-Galacturonat | *Aspergillus niger* | *Quelle: Tokyo Chemical Industry (TCI)* - *Produkt P0026* |

### Analytische Methoden

### High Performance Anion Exchange Chromatography

Zur Quantifizierung von D-Galacturonsäure/D-Galacturonat und Galactarsäure/Galactarat mittels HPAEC (High Performance Anion Exchange Chromatography) wurde ein Dionex ICS6000 System mit AS-AP Autosampler verwendet. Die Messung erfolgte mittels Leitfähigkeitsdetektion (CD) gekoppelt an einen Dionex AERS 500 elektrolytisch regenerierten Suppressor im externen Wassermodus. Zur Trennung der Analyten wurde eine Dionex IonPac AS11-HC-4µm Säule mit entsprechender Vorsäule sowie einem NaOH-Gradienten verwendet. Das Laufmittel wurde zusätzlich mit einer Dionex ATC (Anion Trap Column) vorbehandelt.

Zur Quantifizierung von L-Arabinose, D-Glucose, D-Galactose, L-Rhamnose und D-Xylose mittels HPAEC (High Performance Anion Exchange Chromatography) wurde ein Dionex ICS6000 System mit AS-AP Autosampler verwendet. Zur Trennung der Analyten wurde eine Dionex CarboPac PA20-fast-4µm Säule mit entsprechender Vorsäule sowie einem NaOH-Gradienten verwendet. Die Analyten wurden mittels gepulsten amperometrischen Detektors (PAD, Gold-Elektrode) und der Wellenform "Carbo, Quad" detektiert.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung bzw. der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bio-one Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH bzw. NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67·10⁻⁸ kat).

Mit den nachfolgenden Beispielen werden bevorzugte Varianten des erfindungsgemäßen Verfahrens noch näher beschrieben. Die in diesen Beispielen eingesetzten Lysate wurden nach den oben beschriebenen Verfahren hergestellt.

### Beispiel 1

### Selektive Oxidation von D-Galacturonat zu Galactarat in Gegenwart von Zuckern

In zwei 2 ml Glas-Vials (Vials I und II) wurden folgende Komponenten vermischt: 93,1 µl deionisiertes Wasser, 125 µl eines 1 M TEA-Puffers (pH 8), 250 µl einer Zuckermischung* (99 g/l L-Arabinose, 100 g/l D-Galacturonsäure, 100 g/l D-Glucose, 24 g/l D-Galactose, 11 g/l L-Rhamnose, 10 g/l D-Xylose; gestellt auf pH 8) sowie 5 µl einer 10 mM NAD⁺-Lösung.

Zum Start der Reaktion wurden zu beiden Ansätzen 2 U NADH-Oxidase I-Lysat sowie 5 U UDH I-Lysat zu Vial I und 1,1 U UDH II-Lysat (gleiche Biomasse-Menge wie UDH I) zu Vial II zugefügt. Die Ansätze wurden unter kontinuierlichem Schütteln (Eppendorf-Thermomixer; 30 °C, 1200 rpm) für insgesamt 20 h inkubiert.

Zur Analyse wurden 50 µl eines Ansatzes mit 200 µl Methanol versetzt und im Eppendorf Thermomixer bei 60 °C und 1200 rpm für 15 min inkubiert. Die Probe wurde in einer Zentrifuge kurz zentrifugiert, mit 750 µl deionisiertem Wasser versetzt, gevortext, für weitere 10 min bei 60 °C und 1200 rpm inkubiert und anschließend für 5 min bei max. g zentrifugiert. Der Überstand wurde 1:400 mit Reinstwasser verdünnt und auf der HPAEC (Leitfähigkeitsdetektion) vermessen. Für die Zuckerbestimmung mittels HPAEC (PAD) wurde der Überstand 1:1600 verdünnt.

Auf diese Weise konnten >99% des D-Galacturonats (50 g/l) in beiden Ansätzen zu Galactarat (Vial I: 56,0 g/l; Vial II: 55,0 g/l) umgesetzt werden. Es konnte keine Konzentrationsänderung der Zucker festgestellt werden, da die hier eingesetzten UDHs selektiv D-Galacturonat oxidieren.
*Die hier eingesetzte Zuckermischung entspricht in ihrer Zusammensetzung einer komplett hydrolysierten Zuckerrüben-Pulpe (Micard et al., 1996; Ward et al., 2015).

### Beispiel 2

### Umsetzung von Zitrus-Pektin zu Galactarsäure

Die Reaktion wurde in einem Multifors Tisch-Bioreaktor (Infors AG) durchgeführt. Als Gefäß wurde ein Glasreaktor (Volumen 1 I) mit aufgesetztem Rührwerk, pH-Elektrode und O₂-Sensor verwendet. Die pH-Kontrolle erfolgte durch die Zudosierung von 5M NaOH oder 1M H₂SO₄.

Zu Beginn wurden 3 g Pektinase aus *Aspergillus niger* in 200 ml Wasser und 17,5 ml eines 1 M KPP-Puffers (pH 6) gelöst. Nach und nach wurden 35 g Zitrus-Pektin* in die Lösung eingerührt. Der pH-Wert wurde durch Zugabe von insgesamt 10 ml einer 5 M NaOH nachgestellt. Die Mischung wurde nun in den Reaktor gefüllt, mit 55 ml Wasser versetzt und unter Rühren auf 30 °C gebracht.

Nach 45 min wurden 7 ml einer 10 mM NAD⁺-Lösung, 4 kU NADH-Oxidase I-Lysat sowie 30 ml UDH I-Lysat eingebracht und die Luftzufuhr auf 0,15 l/min eingestellt.

Während des Betriebs wurden laufend Proben von der Reaktorlösung genommen, die folgendermaßen analysiert wurden: 50 µl der Reaktorlösung wurden mit 200 µl Methanol versetzt und im Eppendorf Thermomixer bei 60 °C und 1200 rpm für 20 min inkubiert. Die Probe wurde in einer Zentrifuge kurz zentrifugiert, mit 750 µl deionisiertem Wasser versetzt, gevortext, für weitere 10 min bei 60 °C und 1200 rpm inkubiert und anschließend für 5 min bei max. g zentrifugiert. Der Überstand wurde mittels eines Agilent-Spritzenfilters (PTFE, 0,2 µm) filtriert. Das Filtrat wurde 1:800 mit Reinstwasser verdünnt und mittels HPAEC (Leitfähigkeitsdetektion) vermessen.

Nach 27 h wurden 306 mg Pektinase aus *Aspergillus niger* (gelöst in 10 ml H₂O) zugesetzt.

Nach 44 h wurden 70 g/l Galactarat (entspricht 91% Umsatz der theoretischen D-Galacturonsäure-Menge in Pektin*) sowie 1,7 g/l D-Galacturonat detektiert.

Danach wurde der gesamte Reaktorinhalt für 30 min auf 70 °C erhitzt. Die Enzyme wurden durch Zentrifugation (10 min bei 4500 rpm) entfernt. Der gelbliche, leicht trübe Überstand wurde durch eine Glasfritte P3 unter Anlegen eines Vakuums filtriert. Das Filtrat wurde nun mit 12 M H₂SO₄ angesäuert (pH 1,4) und im Kühlschrank über Nacht inkubiert, wodurch sich ein farbloser Niederschlag bildete. Dieser wurde mittels einer Glasfritte (P3) abfiltriert und über Nacht im Vakuumtrockenschrank bei 50 °C getrocknet. Durch Aufkonzentrieren und Kühlen der Lösung konnten weitere Fraktionen gewonnen werden.

Auf diese Weise konnten 13,2 g Galactarsäure in hoher Reinheit (> 95%) gewonnen werden.
* Anmerkung: Laut Spezifikation für "Pektin from Citrus" von Tokyo Chemical Industry (TCI; Produktnummer: P0024) besteht das Pektin zumindest zu 58% aus D-Galacturonsäure. Enzymatische Hydrolysen mittels Pektinase von *Aspergillus niger* in Vorversuchen zeigten, dass der D-Galacturonsäure-Gehalt bei ca. 70% liegt, was für die Umsatz-Berechnung herangezogen wurde.

### Beispiel 3

### Oxidation von D-Galacturonat zu Galactarat-Vergleich von zwei Regenerationssystemen

In zwei 2 ml Glas-Vials (Vials I und II) wurden folgende Komponenten vermischt: 142,2 µl deionisiertes Wasser (Vial I) bzw. 33,4 µl deionisiertes Wasser (Vial II), 125 µl eines 1 M TEA-Puffers (pH 8), 200 µl einer D-Galacturonsäure-Lösung (250 g/l; gestellt auf pH 8), 5 µl einer 10 mM NAD⁺-Lösung sowie zusätzlich 112 mg Natrium-Pyruvat (entspricht 4 Äquivalente bezogen auf D-Galacturonsäure-Menge) in Vial II.

Zum Start der Reaktion wurden zu beiden Ansätzen 5 U UDH I-Lysat sowie 1 U NADH-Oxidase II-Lysat zu Vial I und 1 U L-Lactat-Dehydrogenase (EC 1.1.1.27; aus Kaninchenmuskel) zu Vial II zugesetzt. Die Ansätze wurden unter kontinuierlichem Schütteln (Eppendorf-Thermomixer; 30 °C, 1200 rpm) für insgesamt 20 h inkubiert.

Zur Analyse wurden 25 µl eines Ansatzes mit 200 µl Methanol versetzt und im Eppendorf Thermomixer bei 60 °C und 1200 rpm für 15 min inkubiert. Die Probe wurde in einer Zentrifuge kurz zentrifugiert, mit 775 µl deionisiertem Wasser versetzt, gevortext, für weitere 10 min bei 60 °C und 1200 rpm inkubiert und anschließend für 5 min bei max. g zentrifugiert. Der Überstand wurde 1:400 mit Reinstwasser verdünnt und auf der HPAEC (Leitfähigkeitsdetektion) vermessen.

Auf diese Weise konnten in Vial I 60% des D-Galacturonats (100 g/l) zu Galactarat (58,9 g/l) und in Vial II 18% des D-Galacturonats (100 g/l) zu Galactarat (21,7 g/l) umgesetzt werden.

Dieses Beispiel zeigt, dass die Kofaktor-Regeneration mittels NADH-Oxidase zu höheren Umsätzen führt als die Kofaktor-Regeneration-Regeneration mittels Lactat-Dehydrogenase (vgl. Chang & Feingold (1969)).

### Literatur

Sakuta, R., & Nakamura, N. (2019). Production of Hexaric Acids from Biomass. International Journal of Molecular Sciences, 20(15), 3660. https://doi.org/10.3390/ijms20153660
Li, X., Wu, D., Lu, T., Yi, G., Su, H., & Zhang, Y. (2014). Highly efficient chemical process to convert mucic acid into adipic acid and DFT studies of the mechanism of the rhenium-catalyzed deoxydehydration. Angewandte Chemie International Edition, 53(16), 4200-4204. https://doi.org/10.1002/anie.201310991
Anet, E. F. L. J., & Reynolds, T. M. (1954). Isolation of Mucic Acid from Fruits. Nature, 174, 930. https://doi.org/10.1038/174930a0
Stark, J. B., Goodban, A. E., & Owens, H. S. (1950). Organic Acids in Sugar Beet Diffusion Juices. Proceedings of the American Society of Sugar Beet Technologists, 6, 578-583. https://bsdf-assbt.org/wpcontent/uploads/2017/12/ASSBTVol6p578to583OrganicAcidsinSugarbeetDiffusionJuices.pdf (aufgerufen am 24.05.2024)
Pigman, W. W., Browning, B. L., McPherson, W. H., Calkins, C. R., & Leaf, R. L. (1949). Oxidation of D-Galactose and Cellulose with Nitric Acid, Nitrous Acid and Nitrogen Oxides. Journal of the American Chemical Society, 71(6), 2200-2204. https://doi.org/10.1021/ja01174a076
van der Klis, F., van Haveren, J., van Es, D. S., & Bitter, J. H. (2017). Synthesis of Furandicarboxylic Acid Esters From Nonfood Feedstocks Without Concomitant Levulinic Acid Formation. ChemSusChem, 10(7), 1460-1468. https://doi.org/10.1002/cssc.201700051
Roman-Benn, A., Contador, C. A., Li, M.-W., Lam, H.-M., Kong, A.-H., Ulloa, P. E., & Ravanal, M. C. (2023). Pectin: An overview of sources, extraction and applications in food products, biomedical, pharmaceutical and environmental issues. Food Chemistry Advances, 2, 100192. https://doi.org/10.1016/j.focha.2023.100192
Kuivanen, J., Biz, A., & Richard, P. (2019). Microbial hexuronate catabolism in biotechnology. AMB Express, 9, 16. https://doi.org/10.1186/s13568-019-0737-1
Rautiainen, S., Lehtinen, P., Chen, J., Vehkamäki, M., Niemelä, K., Leskelä, M., & Repo, T. (2015). Selective oxidation of uronic acids into aldaric acids over gold catalyst. RSC Advances, 5(25), 19502-19507. https://doi.org/10.1039/C5RA01802A
van der Klis, F., Gootjes, L., van Haveren, J., van Es, D. S., & Bitter, J. H. (2018). From batch to continuous: Au-catalysed oxidation of D-galacturonic acid in a packed bed plug flow reactor under alkaline conditions. Reaction Chemistry & Engineering, 3(4), 540-549. https://doi.org/10.1039/C8RE00047F
van der Klis, F., Frissen, A. E., van Haveren, J., & van Es, D. S. (2013). Waste Not, Want Not: Mild and Selective Catalytic Oxidation of Uronic Acids. ChemSusChem, 6(9), 1640-1645. https://doi.org/10.1002/cssc.201300367
Mojzita, D., Wiebe, M., Hilditch, S., Boer, H., Penttilä, M., & Richard, P. (2010). Metabolic Engineering of Fungal Strains for Conversion of D-Galacturonate to meso-Galactarate. Applied and Environmental Microbiology, 76(1), 169-175. https://doi.org/10.1128/AEM.02273-09
Paasikallio, T., Huuskonen, A., & Wiebe, M. G. (2017). Scaling up and scaling down the production of galactaric acid from pectin using Trichoderma reesei. Microbial Cell Factories, 16, 119. https://doi.org/10.1186/s12934-017-0736-3
Tamminen, A., Turunen, R., Barth, D., Vidgren, V., & Wiebe, M. G. (2022). Use of ambr®250 to assess mucic acid production in fed-batch cultures of a marine Trichoderma sp. D-221704. AMB Express, 12, 90. https://doi.org/10.1186/s13568-022-01436-4
Protzko, R. J., Latimer, L. N., Martinho, Z., de Reus, E., Seibert, T., Benz, J. P., & Dueber, J. E. (2018). Engineering Saccharomyces cerevisiae for co-utilization of D-galacturonic acid and D-glucose from citrus peel waste. Nature Communications, 9, 5029. https://doi.org/10.1038/s41467-018-07589-w
Vidgren, V., Halinen, S., Tamminen, A., Olenius, S., & Wiebe, M. G. (2020). Engineering marine fungi for conversion of D-galacturonic acid to mucic acid. Microbial Cell Factories, 19, 156. https://doi.org/10.1186/s12934-020-01411-3
Wagner, G., & Hollmann, S. (1976). A new enzymatic method for the determination of free and conjugated glucuronic acid. Journal of Clinical Chemistry and Clinical Biochemistry, 14(5), 225-226. https://doi.org/10.1515/cclm.1976.14.1-12.225
Chang, Y. F., & Feingold, D. S. (1969). Hexuronic Acid Dehydrogenase of Agrobacterium tumefaciens. Journal of Bacteriology, 99(3), 667-673. https://doi.org/10.1128/jb.99.3.667-673.1969
Su, H.-H., Guo, Z.-W., Wu, X.-L., Xu, P., Li, N., Zong, M.-H., & Lou, W.-Y. (2019). Efficient Bioconversion of Sucrose to High-Value-Added Glucaric Acid by In Vitro Metabolic Engineering. ChemSusChem, 12(10), 2278-2285. https://doi.org/10.1002/cssc.201900185
Petroll, K., Care, A., Bergquist, P. L., & Sunna, A. (2020). A novel framework for the cell-free enzymatic production of glucaric acid. Metabolic Engineering, 57, 162-173. https://doi.org/10.1016/j.ymben.2019.11.003
Sakuta, R., Takeda, K., Igarashi, K., Ohno, H., & Nakamura, N. (2016). Pyrroloquinoline quinonedependent glucose dehydrogenase anode: D-Galacturonic acid oxidation and galactaric acid production. Journal of Molecular Catalysis B: Enzymatic, 133(1), S76-S79. https://doi.org/10.1016/j.molcatb.2016.11.021
Nakagawa, T., Abe, H., Gessei, T., Takeda, K., Igarashi, K., & Nakamura, N. (2022). Biorefinery of galacturonic acid using a biofuel cell as a reactor. Reaction Chemistry & Engineering, 7(12), 2629-2635. https://doi.org/10.1039/D2RE00202G
Vastano, M., Pellis, A., Botelho Machado, C., Simister, R., McQueen-Mason, S. J., Farmer, T. J., & Gomez, L. D. (2019). Sustainable Galactarate-Based Polymers: Multi-Enzymatic Production of Pectin-Derived Polyesters. Macromolecular Rapid Communications, 40(22), 1900361. https://doi.org/10.1002/marc.201900361
Kobayashi, M., Nishihara, H., & Kobayashi, S. (1999). Oxidation of Uronic Acids by a Large Excess of Glucose Oxidase Preparations. Journal of Applied Glycoscience, 46(1), 1-7. https://doi.org/10.5458/jag.46.1
Riov, J. (1975). Metabolism of Uronic Acids in Plant Tissues: Partial Purification and Properties of Uronic Acid Oxidase from Citrus Leaves. Plant Physiology, 55(4), 602-606. https://doi.org/10.1104/pp.55.4.602
Boverio, A., van Beek, H. L., Savino, S., Ranoux, A., Huijgen, W. J. J., Raaijmakers, H. W. C., Fraaije, M. W., & Lončar, N. (2023). Biochemical and Structural Characterization of a Uronic Acid Oxidase from Citrus sinensis. ChemCatChem, 15(21), e202300847. https://doi.org/10.1002/cctc.202300847
Lin, B., & Tao, Y. (2017). Whole-cell biocatalysts by design. Microbial Cell Factories, 16, 106. https://doi.org/10.1186/s12934-017-0724-7
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Protein [Internet]. Bethesda (MD): National Library of Medicine (US), National Center for Biotechnology Information. Accession No. WP_134697446.1, NAD(P)-dependent oxidoreductase [Pseudomonas putida]. Verfügbar unter: https://www.ncbi.nlm.nih.gov/protein/WP_134697446.1 (Zugriff am 27.05.2024)
Yoon, S.-H., Moon, T. S., Iranpour, P., Lanza, A. M., & Prather, K. J. (2009). Cloning and Characterization of Uronate Dehydrogenases from Two Pseudomonads and Agrobacterium tumefaciens Strain C58. Journal of Bacteriology, 191(5), 1565-1573. https://doi.org/10.1128/jb.00586-08
Protein [Internet]. Bethesda (MD): National Library of Medicine (US), National Center for Biotechnology Information. Accession No. WP_003989401.1, NAD(P)-dependent oxidoreductase [Streptomyces viridochromogenes]. Verfügbar unter: https://www.ncbi.nlm.nih.gov/protein/WP_003989401.1 (Zugriff am 27.05.2024)
Pick, A., Schmid, J., & Sieber, V. (2015). Characterization of uronate dehydrogenases catalysing the initial step in an oxidative pathway. Microbial Biotechnology, 8(4), 633-643. https://doi.org/10.1111/1751-7915.12265
Matsumoto, J., Higuchi, M., Shimada, M., Yamamoto, Y., & Kamio, Y. (1996). Molecular Cloning and Sequence Analysis of the Gene Encoding the H2O-forming NADH Oxidase from Streptococcus mutans. Bioscience, Biotechnology, and Biochemistry, 60(1), 39-43. https://doi.org/10.1271/bbb.60.39
Micard, V., Renard, G. M. G. C., & Thibault, J.-F. (1996). Enzymatic saccharification of sugar-beet pulp. Enzyme and Microbial Technology, 19(3), 162-170. https://doi.org/10.1016/0141-0229(95)00224-3
Ward, D. P., Cärdenas-Fernändez, M., Hewitson, P., Ignatova, S., & Lye, G. J. (2015). Centrifugal partition chromatography in a biorefinery context: Separation of monosaccharides from hydrolysed sugar beet pulp. Journal of Chromatography A, 1411, 84-91. https://doi.org/10.1016/j.chroma.2015.08.006

## Patentansprüche

1. Verfahren zur Herstellung von Galactarsäure, indem Galacturonsäure in einer wässerigen Lösung *in vitro* mit einer Dehydrogenase mit NAD(P)⁺ als Kofaktor unter Bildung von reduziertem Kofaktor NAD(P)H oxidiert wird, **dadurch gekennzeichnet, dass** der reduzierte Kofaktor NAD(P)H mit einer NAD(P)H-Oxidase oxidiert wird.

2. Verfahren nach Anspruch 1, **dadurch gekennzeichnet, dass** als Dehydrogenase eine Uronsäure/Uronat-Dehydrogenase eingesetzt wird.

3. Verfahren nach Anspruch 1, **dadurch gekennzeichnet, dass** die NAD(P)H-Oxidase zur Oxidation von NAD(P)H zu NAD(P)⁺ eine Aminosäuresequenz umfasst, die ausgewählt ist aus der Gruppe bestehend aus:
i) einer Aminosäuresequenz, die eine Identität zu SEQ ID Nr. 2 oder SEQ ID Nr. 4 von mindestens 80% aufweist,
ii) einer Aminosäuresequenz, die durch eine Nukleinsäure kodiert wird, die eine Identität zu SEQ ID Nr. 1 oder SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 oder SEQ ID Nr. 3 bindet.

4. Verwendung einer NAD(P)H-Oxidase zur Oxidation von NAD(P)H zu NAD(P)⁺, welche eine Aminosäuresequenz umfasst oder daraus besteht, die ausgewählt ist aus der Gruppe bestehend aus:
i) einer Aminosäuresequenz, die eine Identität zu SEQ ID Nr. 2 oder SEQ ID Nr. 4 von mindestens 80% aufweist,
ii) einer Aminosäuresequenz, die durch eine Nukleinsäure kodiert wird, die eine Identität zu SEQ ID Nr. 1 oder SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 oder SEQ ID Nr. 3 bindet.
